# EUROPEAN PATENT APPLICATION

(11) **EP 1 782 839 A1**
(43) Date of publication of application: **09.05.2007**
(21) Application number: 05425773.8
(22) Date of filing: 03.11.2005
(51) Int. Cl.: A61L 2/00, A61K 31/56, A61K 9/14

(54) **Sterilization process of Glucocorticosteroid by supercritical CO2**

(71) Applicant: Genetic S.p.A., 84083 Castel San Giorgio (SA) (IT)
(72) Inventor: Pavese, Carmelo Rocco, 84083 Castel san Giorgio (SA) (IT)

(57) **Abstract**

The process of sterilization consists of using glucocorticosteroids, particularly Beclomethasone dipropionate, e/o others substances with a non superior diameter to 8 - 9 microns in double material envelope plastic fit for the sterilization vapor and to gas to the temperature of 125 ±s 1°C in autoclave and/or in system for the treatment of substances with supercritical fluid to a pressure of 80 bar with supercritical CO2 for a time of 25 minutes. Under such conditions a sterile product is gotten. In the test of inoculate, the procedure results able to effectively eliminate the indicative microorganisms foreseen by PH. Eur. After the sterilization new products of degradation and the initial impurities are not formed they don't increase, and/or are in the parameters accepted by the PH. Eur.

## Description

### Technical field

This invention, concerns a sterilization process of glucocorticosteroids in dried powder form with low water content. The interested glucocorticosteroids to this process are all, but particularly the anti asthmatics used by nasal or oral inhalation and the ones that have an anti inflammatory activity (Budesonide, Triamcinolone Acetonide, Fluticasone, Triamcinolone Diacetate,Triamcinolone Hexacetonide, Metilprednisolone Sodium Succinate, Metilprednisolone Acetate, Beclomethasone Dipropionate, Momethasone Furoate, Idrocortisone Acetato, Medrossiprogesterone Acetate).

### State of technique

It is known that the sterility, is an important requisite for the pharmaceutical formulations to be used by inhalation through solutions or suspensions, distributed like pressurized aerosol (MDI) or by nebulization (NEBUL) using opportune ultrasound instruments or through compressed air.

Regarding the glucocorticosteroids, there are known various process and methods of sterilization, each one presents some deficiency or some limitations. In general terms, it's effected the pre sterilization of the active principle by dried heating or by radiation followed by preparation of the formulation in asepsis or using another method that first foresees the formulation which is subsequently submitted to sterilization by treatment in autoclave. The methods of pre sterilization, require a following mixing phase of the active principle with the others components of the formulation then, they also require to dispose the final formulation in asepsis; this behaves the disadvantage that doesn't consent to sterilize immediately the final formulation first of distributing it in the final sterile container.

The standard treatments of autoclaving, in case of watery suspensions of corticosteroids thermal weak, don't result suitable because they generate the degradation of the active principle and they can also generate a reaggregation of particles, that belongs to the active principle, which are also difficult to separate and disperse in the suspension, such to jeopardize the therapeutic efficiency especially in case of aerosol therapy.

The sterilizing filtration, in case of the suspensions, is little practicable because it's requested the use of filters with a dimension of the pores not superior to 0,2 micron, under the diameter of the most particles present in the active principle, so most of them are blocked by the filter.

The sterilization through radiation, essentially gamma rays, have a drawback that determinate significant alterations in the raw material with a total increasing of the degradation products and the formation of individuals new products deriving from the degradation.

The sterilization, in organic solvents and crystallization, gives problems to eliminate all the surpluses solvents (ethanol, methanol, isopropanole, ethyl acetate and others) in the final product.

A process of sterilization of the glucocorticosteroid, uses a cold mixture of ethylene oxide and carbon dioxide ( PT-to-69652 ). In the patent examples of sterilization of glucocorticosteroids are brought, what the Prednacindone, Dexamethasone, Prednsolone and relative ester, fluorine derived e salt, including desamethasone Acetate, Desamethasone phosphate, Prednisolone pivalate and 9-alfafluoroprednisolone. A fundamental limitation of this process is that the oxide of ethylene is toxic and the residue that remains in the product after the sterilization of the glucocorticosteroid it is elevated and not conforming to the guide lines pharmaceutical (ICH), that require a very low residue of oxide of ethylene in the ended product. Therefore to the light of the in demand actual regolamentations the method would not result fit for the production of acceptable pharmaceutical formulation of glucocoticosteroids.

An other sterilization technique of the glucocorticosteroids is that of the sterilization in heater with dry heat.

Such technique has been described in the patent AstraZeneca USA 6, 392, 036 and modified in patent EP 04425835.8-2113 ( Genetic spa ).

Astra patent disclosure that the sterilization of micronized glucocorticosteroids it's effected through temperatures between 100°C and 130°C for 1-10 hours, according to the temperature and the glucocorticosteroid.

The glucocorticosteroid is polishedly divided in particles, with a median mass inferior to 10 or 5 micron, and it's essentially dry (water content inferior to 1 , 0.5 or 0.3).

Before the sterilization, Bioburden it's preferentially inferior to 1. CFU/g. The process can be applied to: budesonide, rofleponide, rofleponide palmitatebetamethasone, fluticasone (propionate), tipredane, desamethasone, beclomethasone (dipropionate), prednisolone, fluocinolone, triamcinolone (acetonide), momethasone (furoate), flumethasone, flunisolide, ciclesonide, deflazacort, cortivazol and others.

With this process it's obtained a sterile product, as defined by Ph. Eur. , USP, BP and others Pharmacopoeias. In the inoculate test, the procedure resulted able to eliminate the indicative microorganisms foreseen by the Ph. Eur.

The process doesn't cause budesonide degradation.

The Genetic patent EP 04425835.8-2113 is a modification of the Astra patent that foresees a reduction of the time of sterilization inclusive among 85-101 minutes and a raising of the range of the temperature of sterilization inclusive among 131° - 141°C. Also with this procedure it is possible sterilize a large number of glucocorticosteroids.

These procedures on climbs industrialist foresee a validation work of the process and of the sterilization heater much gives a complex, in how much be necessary show that the temperature of sterilization is reached in every point of the product and maintained for the necessary time.

Considering the good results of this process, an improvement has been thought to let it be more simple and effective, able to mitigate the drawbacks listed in other types of sterilization process used for the production of a sterile suspension of glucocorticosteroids.

Is then thought to use the supercritical carbon dioxide for the sterilization of the glucocorticosteroid.

How is known the supercritical carbon dioxide has been used in place of the volatile organic solvents to extract active components from the natural products and even for the micronization of pharmaceutical substance.

USA patent 6,468,994 disclose a preparation method of thin particles, with the use of CO2 supercritical introducing for first a solution of budesonide in an organic solvent. The process uses of a described apparatus in WO 95/01221.

USA patent 6,461,642 disclose a method for the obtaining of crystalline particle using supercritical fluid and subcritical, dissolving the substance of departure in a first solvent, introducing the solution in an apparatus together with the supercritical or subcritical fluid.

A method of preparation of microparticles of Budesonide using supercritical fluid is described from : Martin TM, Bandi N, Shulz R, Roberts CB, Kompella UB. In AAPS PharmSciTech. 2002; 3(3); article 18.

Recently has begun to use the CO2 supercritical even for the sterilization of prepared foodstuffses.

Spilimbergo, S., Bertucco, A., Lauro, F.M., Bertoloni, G. (2003). " Inactivation of bacillus Subtilis spores by supercritical CO2 treatment or conventional heats". Innovative Food Sci. and Emerging Technologies. 4: 161-165.

### Description of the invention

How is known the carbon dioxide, to the temperature of 37, 7 °c and pressure of 68 bar, finds in the supercritical state, when not there is distinction among vapor phase and liquid phase. Increasing the temperature to constant pressure ( 68 bar), and viceversa increasing the pressure to constant temperature ( 37,7 °C) the CO₂ remains in the supercritical state. They are possible then various pressure and temperature combinations that influence, varying themself, the ownership solubilizes and sterilize of the supercritical dioxide carbon. The sterilization in accordance with the present invention happens in autoclave with times of sterilization inclusive among 15 and 60 minutes, in a temperature interval and inclusive pressure respectively among 80° and 135°C and among 70 and 150 bar pressure. Under these conditions the supercritical gas is to intimate contact with the product reaching in every point the desired temperature of sterilization.

In a preferred formality the process of sterilization happens to a temperature of 125°±1°C for a time of 25 minutes to the pressure of CO₂ of 80 bar.

The process of sterilization can be conducted in saturated environment filled with supercritical carbon dioxide, or fluxing supercritical carbon dioxide in the room of sterilization to the temperature and pressure condition and in the times above brought.

Surprisingly the glucocorticosteroid treated with supercritical carbon dioxide, in the described conditions by this patents, doesn't suffer significant degradations, maintaining the same profile of purity before and after the treatment.

The used autoclave is constituted by a cylinder in steel capable of withstand to inclusive pressures among the 100 and the 200 bar. The used carbon dioxide in the process, stored in liquid phase in opportune cylinders, comes at first filtered sterilely in liquid phase on filter absolute from 0.2 micron ( under pressure and to a temperature less than that of the supercritical point ) and subsequently course in supercritical phase for heating to the sterilization temperature of 125°C.

The carbon dioxide, heated to the sterilization temperature, is made to flux inside the room, saturating it, and with it also saturating the product to sterilize. The temperature and the pressure of sterilization comes recording in the exit point of the carbon dioxide from the autoclave.

The starting glucocorticosteroid material, in polishedly divided form, it's essentially dried, contains less than 1% W/W of water.

Preferably contains less than 0.5% of water and is manufactured in double envelope of plastic fit for the vapor sterilization or to gas. The starting material used for the process have the bioburden inferior of 10 CFU (colonies forming unities) per gram or preferably inferior to 1 CFU/g.

The sterilized glucocorticosteroid, with the present process, will essentially have the same pharmacological activity, the same psychical and chemical properties regarding physical form and purity of the starting material, with which it was prepared; especially the chemical degradation, caused by the present sterilization method, will be limited.

The glucocorticosteroid of the invention it's preferably at least 98,5% in pure weight, preferably pure at least 99% in weight.

It happened that in the glucocorticosteroid, after the sterilization, haven't grown new degradation products and the initial impurities, before the process, haven't increased, and/or re entered in the parameters accepted by the European Pharmacopoeia and with a lessening regarding the quantity of active principle not very meaningful.

The verify procedure consists in the determination title and impurity with corroborated methods of the departure material and to perform subsequently the same controls on the sterilized product. They come besides compared, before and after sterilization, the other chemical-physical parameters what: the crystalline form ( x-Ray ), the granulometric distribution, the contain of water (Karl -Fisher), the sulphuric ashes, the fusion point, the rotatory power and the residual solvents.

We Bring as example the corresponded analytical values on the Beclomethasone Dipropionate before and after the sterilization with supercritical carbon dioxide to 125°C±1° for 25 minutes to the pressure of 80 bar.

**TAB.1**

| Beclomethasone Dipropionate | | | |
|---|---|---|---|
| Type TEST | Specifications | Product before | Product sterilized |
| Aspect | Powder white or at least white crystalline; | conform | conform |
| Solubility | little soluble in water, freely soluble in acetone, very soluble in chloroform. | conform | conform |
| Identification | Positive (HPLC) | positive | conform |
| Sulphric ashes | ≤ 0.1% | negligibile | conform |
| Contained of water (K.Fischer) | ≤ 0.5% | 0.30 | 0.28 |
| | | | |

| Correlated substances | | | |
|---|---|---|---|
| Beclomethasone | ≤0.1% | negligibile | negligibile |
| Beclomethasone 17-propionate | ≤0.1% | <0.02 | <0.02 |
| Beclomethasone 21-propionate | ≤0.3% | 0.09 | 0.09 |
| Beclomethasone 17-propionate 21-acetate | ≤0.1% | 0.03 | 0.03 |
| 6alfa-bromine analogous | ≤0.1% | <0.02 | <0.02 |
| Beclomethasone tripioponate | ≤0.1% | <0.01 | <0.01 |
| Single impurity unknow | ≤0.10% | 0.03 | 0.04 |
| Total impurities | ≤1.5% | 0.26 | 0.029 |
| | | | |
| Title (HPLC) | 97.0-103% | 99.7 | 99.5 |
| | | | |

| Residual solvents | | | |
|---|---|---|---|
| Methylene chlorinate | ≤ 100 ppm | 6 | negligible |
| Ethyl acetate | ≤ 200 ppm | 49 | 5 |
| Tetrahydrofuran | ≤ 100 ppm | negligible | negligible |
| Pyridine | ≤ 200 ppm | negligible | negligible |

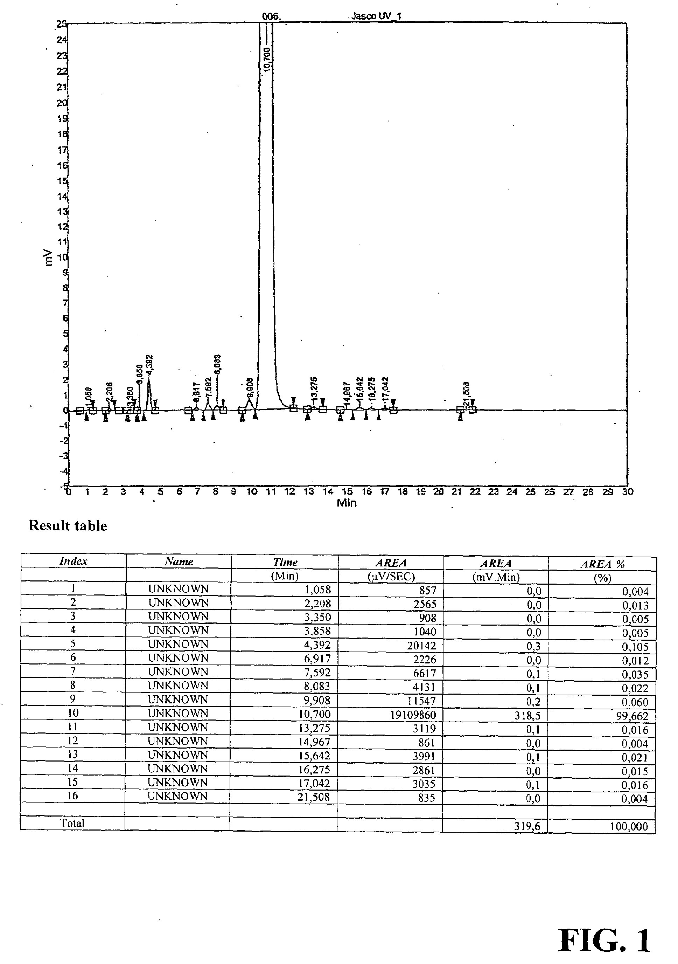

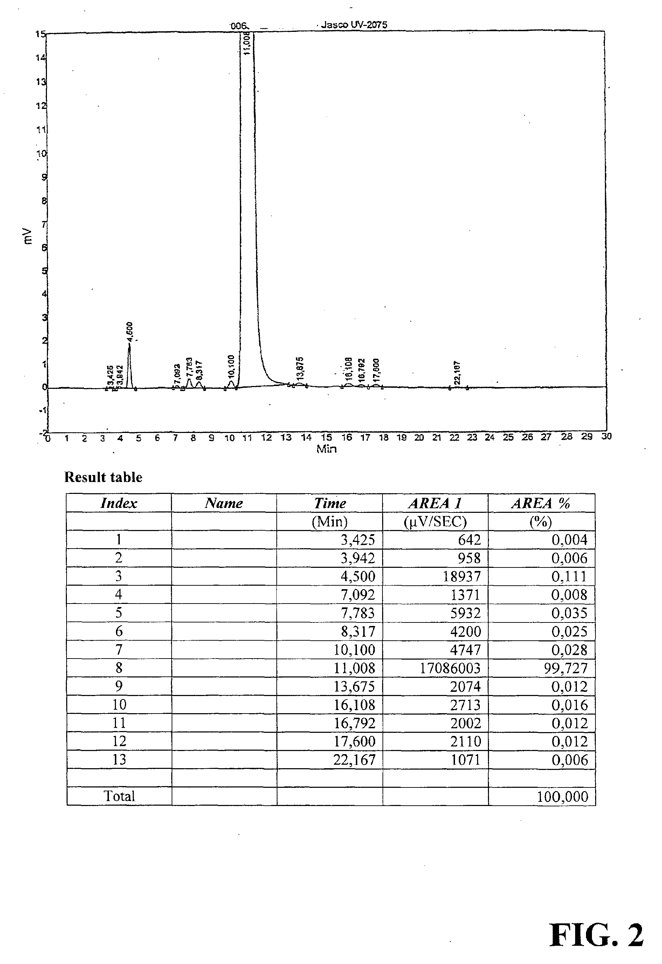

As show tab. 1 and from comparison with the reliables chromatographs fig.1 e fig.2 , after the sterilization, the initial impurities haven't increased, and/or they reenter in the parameters accepted by the European Pharmacopoeia, there isn't degradation with the formation of new products and the quantitative diminution of active principle isn't very meaningful.

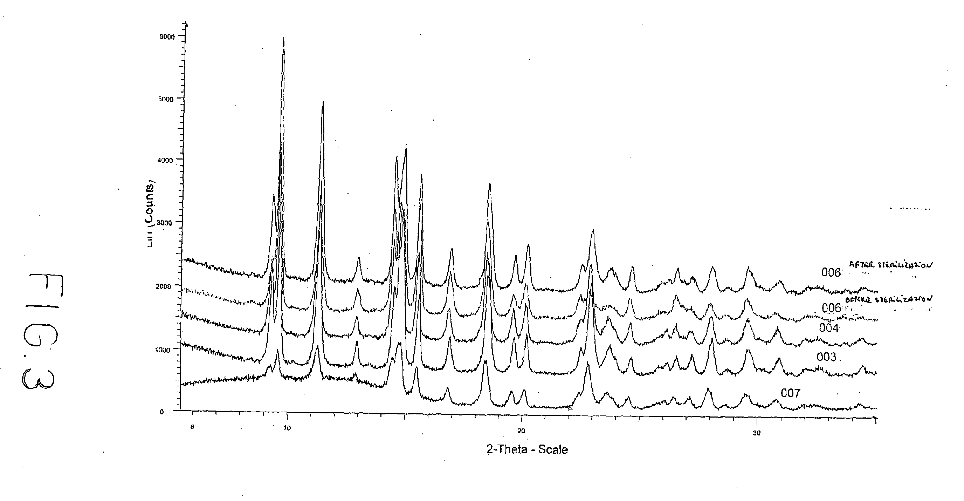

Similarly they remains unchanged, before and after the sterilization, the other physical parameters what the granulometric distribution, the fusion point, the rotatory power and crystalline form, in fig.3 the x -Ray layout for the dipropionate beclomethasone. The only variation that is note is the sensitive diminution of the residual solvents justified by the fact that the supercritical carbon dioxide goes into intimate contact with the crystals of the product and when escapes extracts the residual solvents contained in the product. It same type of treatment with supercritical carbon dioxide has stated performed on other glucocorticosteroids what: Budesonide, Triamcinolone Acetonide, Fluticasone, Triamcinolone Diacetate, Triamcinolone Hexacetonide, Metilprednisolone succinate sodium, Metilprednisolone acetate, Beclomethasone Dipropionato, Momethasone Furoate, acetate hydrocortisone and Medrossiprogesterone acetate.

In all the cases took in consideration not are noticed degradation processes with reduction of the activity of the active principle such to jeopardize the effectiveness of the medicine in conformity to when expectation of the European pharmacopoeia.

After having validated and verified the acceptability of the degradation products and the quantity of active principle before and following the validation, it has been validated the microbiological method to exclude resistance of the microorganisms in the sterilization process.

Initially it occurs the sterilization process effectiveness with the Bacillus Subtilis.

### Procedure

10 quotas of 0.5 g of active principle (Beclomethasone Dipropionate ) are inoculated with 0.1 ml of a B. Subtilis suspension which contains about 10⁷ spores; 9 of this quotas are submitted to the supercritical CO2 sterilization cycle through 125°C+1 °C for 25 minutes and 80 bar;
A quota it's used as control;
On the 9 unities sterilized and on the control unity it's determined the spores population, by counting for inclusion in agar;

The sterilization process it's considered validated when it's registered a reduction regarding the number of the spores 10⁶.

In the following table 2 are reported the results obtained for the singles quotas treated and of control.

In all quotas, submitted to the sterilization with the present method, have been registered a number of spores inferior to 10.

**TAB.2**

| N° identification | Cfu/g | Log | logarithmic reduction |
|---|---|---|---|
| positiv control | 1.1 × 10⁸ | 8.04 | |
| quota 1 | <10 | <1 | > 7.04 |
| quota 2 | <10 | <1 | > 7.04 |
| quota 3 | 10 | 1 | 7.04 |
| quota 4 | <10 | <1 | > 7.04 |
| quota 5 | <10 | <1 | > 7.04 |
| quota 6 | 10 | 1 | 7.04 |
| quota 7 | <10 | <1 | > 7.04 |
| quota 8 | 10 | 1 | 7.04 |
| quota 9 | <10 | <1 | > 7.04 |

Then the efficiency of the sterilizing process has been verified with others microorganisms.

For each microbial log, 6 quotas of 0.5 g of active principle are inoculated with approximately 10² - 10³ of the following microorganisms:
E. Coli, B. Subtilis, S.Typhi, C. Albicans, A. Niger, M. Luteus, S. Epidermidis,
C. Sporogenes, P. Aeruginosa.

Regarding the 6 quotas, 5 are submitted to the sterilization cycle by supercritical CO2 through 125°C+1 for 25 minutes and 80 bar, one quota it's used to determinate the initial microbial population. On the quotas submitted to sterilization it's effected the sterility test.

In a test, it has been gotten the following result:

**TAB. 3**

| Microorganisms | before | after | |
|---|---|---|---|
| E.Coli | 2.7x 10³ | 0 | |
| B.Subtilis | 1.9x 10⁴ | 0 | |
| S.Tiphy | 5.4x 10⁴ | 0 | |
| C.Albicans | 3.2x 10³ | 0 | |
| A.Niger | 2.0x 10³ | 0 | |
| M.Luteus | 2.2x 10⁴ | 0 | |
| S.Epidermidis | 2.9x 10³ | 0 | |
| C.Sporogens | 2.1x 10² | 0 | |
| P.Aeruginosa | 5.1x10⁴ | 0 | |
| | | | |

As we deduce by looking Table 3 budesonide treatment through 125°C + 1°C for 25 minutes and 80 bar pressure allows an effective sterilization for a wide variety of microorganisms.

The same type of test has stated performed for other glucocorticosteroids what: Budesonide, the Triamcinolone Acetonide, the Fluticasone, the Triamcinolone Diacetato, the Triamcinolone Hexacetonide, the Momethasone Furoate, the hydrocortisone Acetate and the Medrossiprogesterone Acetate.
In all the cases the sterile product is gotten also confirming for the others glucorticosteroids the effectiveness of the process of sterilization with supercritical CO2

Examples of application in literature of the CO2 supercritical are found in sterilization processes of pharmaceutical prepared, but with times, pressures and temperatures some distinct from those adopted in the present patent, with good results but equally different in how much to inactivation of the bacteriological microrganism position.

Ishikawa H, Shimoda M, and others. "Inactivation of Bacillus spores by the supercritical carbon dioxide micro-bubble method ". Department of Food Science and Technology, Faculty of Agriculture, Kyushu University, Fukuoka, Japan.
Ratna R. Sharma, Ali Demirci and others ."Supercritical Carbon Dioxide Treatment to Inactivate Aerobic Microorganisms on Alfalfa Seeds" Journal of Food Safety, Volume 21, Number 4.

Angela K. Dillow, Fariba Dehghani, and others. "Bacterial inactivation by using near- and supercritical carbon dioxide " Medical Sciences Vol. 96, Issue 18, 10344-10348.

In the process object of the present invention it is found a combination of preferred values of the temperature, pressure with times extremely foyers of such application to allow a big advantage for the industrial manufacture and allows an effective sterilization for to wide variety of microorganisms.

With the combined action of the select values of the temperature and pressure of the CO2 supercritical, for example 125°C ± 1°C to 80 bar pressure, characteristic to the present invention, is guaranteed a rapid diffusion in the walls of the cell of the microorganism and then the alteration of his PH, that makes more easy the damage of the cells of the microorganism, inactivating them, and besides the increase action intern of the pressure favors, more that in the aforesaid experimentations, the extraction through the walls of the cell of the lipids or the breakup of the his walls, killing the bacterial microorganism.

Then has to hold in account of the fact that is gotten a final product more pure of solvents, in comparison with the initial product.

A different mode to characterize the efficiency of a sterilization process consists in using valour D^{T}, the requested time (in minutes) to reduce (kill) a standard population of spores for 90% (surviving fraction of 1/10 to the specific T temperature in °C).

### Procedure

2 grams of active principle have been inoculated with 1ml of B.Subtilis suspension that contains about 10⁶ spores. The substance and the spores have been mixed and brought to dry for 3 hours at 55°C.

The inoculated active principle has been mixed with 9g. of active principle not inoculated. 5g. of such sample has been submitted to the same sterilization cycle and withdrawn to established time intervals: 2; 4; 6; 8; 10 minutes.

A quota of 1 gram has been used for the determination of the initial contamination.

On all the quotas has been determined the number of spores per gram during time, by the counting for inclusion in agar and then the D^{T}.

In the following table 4 are reported the results obtained after the various sterilization times.

**TAB.4**

| Sterilization time at 125°C±1°C | cfu/ml Count |
|---|---|
| 0 minutes | 1.4x 10⁶ |
| 2 minutes | 1.5x 10¹ |
| 4 minutes | 2.0x 10¹ |
| 6 minutes | 1.9x 10¹ |
| 8 minutes | < 10 |
| 10 minutes | < 10 |
| 25 minutes | Sterile |

We saw how, in the present process, can be obtained a valour D^{T} in less than 25 minutes (higher of two minutes) through the selected temperature T, where T is the interval 125 + 1°C. The sterilization process is conduced so that all the parts can reach and maintain the desired temperature in the wished time.

The present process of sterilization of a containing powder of the powder of the Beta2 agonist and the simple glucocorticosteroid, ester, salt, acetate, relatively to the combination of select values to tall pressure, to the preferred temperature of + 125°C±1°C. it is possible to also use it with others type of supercritical gas.

The process of sterilization it is possible to also use of a powder containing formoterolo, salbutamolo, salmeterolo, ipratropio bromide foresees a treatment with supercritical CO2 understood among a temperature of +80°C and +135°C, to an inclusive pressure between 70 and 150 bar, for a superior period to the 15 minutes and minor of 120 minutes, in double envelope of plastic material fit for the vapor sterilization or gas.

A sterile pharmaceutical formulation by inhalation, contains, preferably, from 0.1 to 5mg/ml of sterilized glucocorticosteroid according to the present process for example budesonide polishdely divided in micro particles where at least 90% have a medium mass diameter less than 20 micron, 80% less than 10 micron 70% less than 7 micron and 60% less than 4 micron, it's prepared by mixing the sterilized glucocorticosteroid sterilized with additional pharmaceutical ingredients for example wetting agents namely surfactants, to obtain an efficient dispersion of glucocorticosteroid particles in the dispersion, used optionally also in combination with lecithin, (in the formulation the surfactant can also have the function of stabilizing agent); PH regulation agents of the suspension, (a formulation used for the inhalation have a PH in the interval between 3.5 and 6, preferably between 4 and 5), initiating agents, agents that make the suspension isotonic to give a stable form to the suspension with a small tendency to form agglomerations or sediments and a condensation agent it's included in the formulation. All the components ca be obtained by sterile filtration from their watery solutions. The resulting sterile suspension can be preserved under pressure in inert and sterile gas, nitrogen or argon and must be filled under antiseptic conditions in a pre sterilized container formed by poly dose or multi dose systems, for example using the blow-fill-seal technology, to obtain a sterile pharmaceutical product.

## Claims

1. Sterilization process of a powder that contains glucocorticosteroid antiasthmatics, anti-allergics and anti-inflammatory, **characterized by** the fact that foresees the treatment of the powder of the β₂ agonist and the glucocorticosteroid simple, ester, salt, acetate with supercritical CO2 to the temperature of 125°C±1°C for 25 minutes and 80 bar of pressure in a dry environment, in double envelope of plastic material fit for the vapor sterilization or to gas.

2. Sterilization process of a powder that contains glucocorticosteroid antiasthmatics, anti-allergics and anti-inflammatory, **characterized by** the fact that the sterilization process of the glucocorticosteroid powder simple, ester, salt, acetate, foresees to treatment with supercritical CO2 understood among a temperature of +80°C +135°C, to an inclusive pressure between 70 and 150 bar, for a period superior to the 15 minutes and minor of 60 minutes, in dry environment and in double envelope of plastic material fit for the vapor sterilization or gas.

3. Process of sterilization of a powder containing formoterolo, salbutamolo, salmeterolo, ipratropio brominate **characterized by** the fact that the process of sterilization of the powder foresees a treatment with supercritical CO2 understood among a temperature of +80°C and +135°C, to an inclusive pressure between 70 and 150 bar, for a superior period to the 15 minutes and minor of 120 minutes, in double envelope of plastic material fit for the vapor sterilization or gas.

4. Sterilization process of a powder that contains glucocorticosteroid and/or β agonisti as to claim 1), and 2) **characterized by** the fact that the substances what simple glucocorticosteroids, salts, ester, acetates, alcohols, that can be sterilized are: Budesonide, Triamcinolone Acetonide, Flutichasone, Triamcinolone Diacetate, Metilprednisolone Sodium Succinate and/or Acetate, Triamcinolone Hexacetonide, Momethasone Furoate, Hydrocortisone Acetate, Medrossiprogesterone Acetate, Betamethasone, Tripedane.

5. Sterilization process of a powder that contains glucocorticosteroids as in claims 1), 2), 3) **characterized by** the fact that glucocorticosteroid before the treatment has a low quantity of water refereed to the medium quantitative of water contained in the mixed glucocorticosteroids for the therapy, less than 05% w/w.

6. Sterilization process of a powder that contains glucocorticosteroids as in claims 1), 2), 3) **characterized by** the fact that the glucocorticosteroid granules have a medium aerodynamic diameter(MAD) not superior than 8-9 micron.

7. Sterilization process of a powder that contains glucocorticosteroids as in claims 1), 2), 3) **characterized by** the fact that the process it's made in an saturated atmosphere with supercritical CO2 with high pressure.

8. Sterilization process of a powder that contains glucocorticosteroids as in claim 1), **characterized by** the fact that the time requested for a reduction not smaller than 90% of the B. SUBTILIS spores population it's essentially higher of 2 minutes, but minor than 25 minutes, through the temperature of 125°C+1°C and 80 bar pressure.

9. Sterilization process of a powder that contains glucocorticosteroids as in claim 1), **characterized by** the fact that the time requested for a reduction to zero of microorganisms population: E. Coli, B. Subtilis, S.Typhi, C. Albicans, A. Niger, M. Luteus, S. Epidermidis, C. Sporogenes, P. Aeruginosa it's 25 minutes through the temperature of 125°C+1°C and 80 bar pressure.

10. Sterilization process of a powder that contains glucocorticosteroids as in claim 1), **characterized by** the fact that the number of spores heat and pressure resistant, specifically B. Subtilis, it's reduced of at least 10⁶ on an inoculated number of 10⁷.
